# EUROPEAN PATENT SPECIFICATION

(11) **EP 1 832 299 B1**
(45) Date of publication and mention of the grant of the patent: **20.07.2011**
(21) Application number: 05809227.1
(22) Date of filing: 24.11.2005
(51) Int. Cl.: A61K 35/34, C12N 9/12

(54) **REMEDY FOR HEART DISEASE USING MAP KINASE TNNI3K**
MITTEL ZUR BEHANDLUNG EINER HERZKRANKHEIT MIT MAP-KINASE-TNNI3K
MEDICAMENT CONTRE UNE MALADIE DU COEUR INCLUANT LA MAP-KINASE TNNI3K

(30) Priority: 24.11.2004 JP 2004338918
(43) Date of publication of application: 12.09.2007
(73) Proprietor: Kumamoto University, Kumamoto-shi Kumamoto 860-8555 (JP)
(72) Inventor: YORINAKA, Hoichi, Kumamoto University, Kumamoto-shi, Kumamoto 8608556 (JP); DING, Jin-Feng, Peking Union Medical College, Beijing 100037 (CN); MENG, Xian-Min, Peking Union Medical College, Beijing 100037 (CN)
(74) Representative: Polypatent
(86) International application number: PCT/JP2005/021535
(87) International publication number: WO 2006/057278

(56) References cited:
- WO-A-03/020912
- MONZEN K ET AL: "Bone morphogenic proteins induce cardiomyocete differentiation through the mitogen-activated protein kinase kinase kinase TAK1 and cardiac transcription factors Csx/Nkx-2.5 and GATA-4" MOLECULAR AND CELLULAR BIOLOGY, AMERICAN SOCIETY FOR MICROBIOLOGY, WASHINGTON, US, vol. 19, no. 10, 1 October 1999 (1999-10-01), pages 7096-7105, XP002958522 ISSN: 0270-7306
- ZHAO YONG ET AL: "CLONING AND CHARACTERIZATION OF A NOVEL CARDIAC-SPECIFIC KINASE THAT INTERFACTS SPECIFICALLY WITH CARDIAC TROPONIN I" JOURNAL OF MOLECULAR MEDICINE, SPRINGER VERLAG, DE, vol. 81, no. 5, 1 January 2003 (2003-01-01), pages 297-304, XP003003866 ISSN: 0946-2716
- LAI Z-F ET AL: 'A NOVEL CARDIAC-SPECIFIC MAP KINASE, TNNI3K, PROMOTES CARDIAC DIFFERENTIATION AND MYOGENESIS IN A PLURIPOTENT P19CL6 CELL MODEL' JOURNAL OF PHARMACOLOGICAL SCIENCES vol. 97, no. SUPPL. I, 01 March 2005, page 128P O-30043, XP003003865
- ZHAO, YONG ET AL.: 'CLONING AND CHARACTERIZATION OF A NOVEL CARDIAC-SPECIFIC KINASE THAT INTERFACTS SPECIFICALLY WITH CARDIAC TROPONIN I' JOURNAL OF MOLECULAR MEDICINE vol. 81, no. 5, 2003, pages 297 - 304, XP003003866
- DATABASE MEDLINE [Online] 2003 LUFT F.C.: 'HEARTS OF THIS ILK RELY ON TNNI3K, A MAPKKK THAT REGULATES TNNIK3', XP003003867 Retrieved from STN Database accession no. (12836637) & JOURNAL OF MOLECULAR MEDICINE vol. 81, no. 5, 2003, pages 279 - 280
- MARIAN A.J. ET AL.: 'THE MOLECULAR GENETIC BASIS FOR HYPERTROPHIC CARDIOMYOPATHY' J. MOL. CARDIOL. vol. 33, 2001, pages 655 - 670, XP002960095
- MONZEN, KOSHIRO ET AL.: 'BONE MORPHOGENETIC PROTEINS INDUCE CARDIOMYOCYTE DIFFERENTIATION THROUGH THE MITOGEN-ACTIVATED PROTEIN KINASE KINASE KINASE TAK1 AND CARDIAC TRANSCRIPTION FACTORS CSX/NKX-2.5 AND GATA-4' MOL. CELL. BIOL. vol. 19, no. 10, 1999, pages 7096 - 7105, XP002958522
- DATABASE CAPLUS [Online] 2003 FIJNVANDRAAT A.C. ET AL: 'TBX5 OVEREXPRESSION STIMULATES DIFFERENTIATION OF CHAMBER MYOCARDIUM IN P19C16 EMBRYONIC CARCINOMA CELLS', XP003003868 Retrieved from STN Database accession no. (2003:770417) & JOURNAL OF MUSCLE RESEARCH vol. 24, no. 2-3, 2003, pages 211 - 218
- DATABASE CAPLUS [Online] 2001 HIROI, YUKIO ET AL.: 'TBX5 ASSOCIATES WITH NKX2-5 AND SYNERGISTICALLY PROMOTES CARDIOMYOCYTE DIFFERENTIATION', XP003003869 Retrieved from STN Database accession no. (2001:510570) & NATURE GENETICS vol. 28, no. 3, 2001, pages 276 - 280

## Description

### Technical Field

The present invention relates to gene therapy technology for heart diseases such as myocardial ischemia or myocardial infarction.

### Background Art

A pluripotent cell line P19CL6 is an internationally effective experimental system for analyzing *in vitro* the differentiation and development of cardiac muscle. The cell was established by Dr. Akemi Ohkubo at Kumamoto University and then developed as a study model for cardiac muscle by Dr. Kazunari Komuro and Dr. Kohshiro Monzen (Habara-Ohkubo A. Differentiation of beating cardiac muscle cells from a derivative of P19 embryonal carcinoma cells. Cell Struct Funct.†1996; 21: 101-110, and Monzen K, Shiojima I, Hiroi Y, Kudoh S, Oka T, Takimoto E, Hayashi D, Hosoda T, Habara-Ohkubo A, Nakaoka T, Fujita T, Yazaki Y, Komuro I. Bone morphogenetic proteins induce cardiomyocyte differentiation through the mitogen-activated protein kinase kinase kinase TAK1 and cardiac transcription factors Csx/Nkx-2.5 and GATA-4. Mol. Cell. Biol.†1999; 19: 7096-7105). The present inventors have observed autonomous action potentials and Ca, Na, and K currents corresponding to differentiation stages in the P19CL6-derived myocardial cells.

TNNI3K is one of the MAP kinases that are specifically and continuously expressed in cardiac muscle, from the newborn to the adult stage. The TNNI3K molecule is characterized by having an amino acid sequence that has homology of approximately 50% with that of ILK (integrin-linked kinase) that is MAPKKK. The amino acid sequence of TNNI3K contains 835 amino acids and has a molecular weight of 93 kD (Zhao, Yong; Meng, Xian-Min; Wei, Ying-Jie; Zhao, Xiu-Wen; Liu, Dong-Qing; Cao, Hui-Qing; Liew, Choong-Chin; Ding, Jin-Feng. Cloning and characterization of a novel cardiac-specific kinase that interacts specifically with cardiac troponin I. Journal of Molecular Medicine (Heidelberg, Germany) (2003), 81(5), 297-304. CODEN: JMLME8ISSN: 0946-2716. CAN 139: 241022 AN 2003: 380646 CAPLUS3. Bibliographic Information). TNNI3K has been assumed to specifically bind to myocardial troponin I so as to regulate myocardial contractions or cardiac functions, but this has not been verified.

The document WO 03/020912 A1 discloses CARK (TNNI3K) molecules that may modulate cell behaviour and act as targets and therapeutic agents for controlling cardiac cell proliferation, differentiation, hypertrophy and migration. CARK nucleic acid and/or protein expression or activity can be used in diagnostic and prognostic assays to detect and/or to treat cardiovascular disorders, e.g. coronary artery disease, ischemia reperfusion and myocardial infarction.

### Disclosure of the Invention

### Object to be Achieved by the Invention

Heart disease such as myocardial ischemia or myocardial infarction is common disease in industrialized nations. There have been few ischemic heart disease cases in Japan compared with the number of such cases in countries in Europe and the United States. With the increase in the elderly population in Japan, the number of patients with ischemic heart disease is increasing. Now ischemic heart disease is one of the three major causes of death in Japan. Treatment for heart disease such as myocardial infarction, and particularly, therapeutic methods using molecules or genes involved in cardiac functions, are currently attracting attention. Although such methods represent very important issues, existing therapeutic methods for such heart disease are insufficient at the present stage. Hence, the development of a novel therapeutic method therefor is desired. Specifically, an object to be achieved by the present invention is to provide a therapeutic means for heart disease such as myocardial ischemia or myocardial infarction.

### Means for Attaining the Object

TNNI3K is a novel MAP kinase that is specifically expressed in cardiac muscle. It has been demonstrated that expression of TNN13K at high levels in myocardial progenitor cells promotes myocardial cell differentiation. Furthermore, it has been confirmed that implantation of myocardial progenitor cells expressing TNN13K at high 2 levels in a rat infarcted myocardial location has effects of promoting regeneration of the affected myocardial cells, decreasing area of infarction, and improving left ventricular remodeling. Based on the above results, it has been revealed that MAP kinase TNNI3K can be used as a target for treating heart disease such as myocardial ischemia or myocardial infarction. The present invention has been completed based on these findings.

The present invention provides a pharmaceutical composition for the treatment of myocardial ischemia or myocardial infarction, which comprises a myocardial progenitor cell expressing MAP kinase TNNI3K at a high level as an active ingredient, wherein said myocardial progenitor cell is a myocardial progenitor cell into which an expression vector for expressing MAP kinase TNNI3K at a high level is introduced, with the provision that human embryonic cells are excluded.

In a preferred embodiment of the pharmaceutical composition the myocardial progenitor cell is an undifferentiated myocardial cell.

In a further preferred embodiment of the pharmaceutical composition the myocardial progenitor cell is an undifferentiated P19CL6 cell.

In a still further preferred embodiment the pharmaceutical composition is for administration to a myocardial location with ischemia or infarction.

### Preferred Embodiments of the Invention

The present invention will be further described in detail.

Unlike conventional cases using ES cells or bone marrow stem cells, treatment according to the present invention involves implanting myocardial progenitor cells caused to express TNNI3K into a location affected with myocardial ischemia or myocardial infarction. Furthermore, measurement of TNNI3K expression levels can also be used as a means for determining therapeutic effects on and prognosis of heart disease such as myocardial ischemia or myocardial infarction.

Myocardial progenitor cells expressing MAP kinase TNNI3K at high levels can be prepared by introducing an expression vector expressing MAP kinase TNNI3K at a high level into myocardial progenitor cells, for example. Such an expression vector expressing MAP kinase TNNI3K at a high level can be constructed by incorporating a gene encoding MAP kinase TNNI3K into an appropriate expression vector. The gene encoding MAP kinase TNNI3K is known and is described in the Journal of Molecular Medicine (Heidelberg, Germany) (2003), 81(5), 297-304 (Gen Bank accession number AF116826), for example. The expression vector to be used in the present invention is not particularly limited, as long as it enables expression of MAP kinase TNNI3K in myocardial progenitor cells. Expression vectors for general animal cells can be used. Specific examples of expression vectors for animal cells include pcDNA6, pcDM8 (Funakoshi Corporation), pcDNAI/AmP (Invitrogen), and pREP4 (Invitrogen).

An expression vector generally contains a promoter. Any promoter can be used herein, as long as expression can be performed in animal cells. Examples of such promoter include a Cytomegalovirus (human CMV) IE (immediate early) gene promoter, an SV40 early promoter, a retrovirus promoter, a metallothionein promoter, a heat shock promoter, and an SRα promoter. In addition, a human CMV IE gene enhancer can also be used with a promoter.

As a method for introducing an expression vector into myocardial progenitor cells, an electroporation method, a calcium phosphate method, a lipofection method, or the like can be used.

Cells obtained by the above method through introduction of an expression vector expressing MAP kinase TNNI3K at a high level into myocardial progenitor cells can generally express MAP kinase TNNI3K at high levels. The expression levels of MAP kinase TNNI3K in the cells can be confirmed by a method such as Northern blot or RT-PCR at the mRNA level or by a method such as Western blot at the protein level.

In the present invention, myocardial progenitor cells are preferably undifferentiated myocardial cells. As undifferentiated myocardial cells, cells derived from mammals (e.g., human, rat, mouse, guinea pig, rabbit, sheep, pig, cattle, horse, cat, dog, and monkey) can be used. For example, undifferentiated P19CL6 cells can be used. P19CL6 cells can be obtained from the RIKEN Bioresource Center. [URL: http://ww2.brc.riken.jp/lab/cell/search.php] with registration No. RCB1539 (Cell name: P19,CL6), for example.

The therapeutic agent for heart diseases of the present invention can be safely used as a therapeutic agent for various types of heart diseases (e.g., cardiac ischemia, myocardial infarction, congestive cardiomyopathy, hypertrophic obstructive cardiomyopathy, hypertrophic non-obstructive cardiomyopathy, idiopathic cardiomyopathy, and chronic heart failure). Specifically, the therapeutic agent for heart diseases of the present invention can be used as an agent for cardiac myogenesis, a heart activator, an agent for regulating pulsation, an agent for enhancing cardiac functions, a heart regeneration agent, or the like.

The therapeutic agent for heart diseases of the present invention can be prepared by suspending myocardial progenitor cells expressing MAP kinase TNNI3K at high levels according to a known method. The thus obtained therapeutic agent for heart diseases can be administered to a mammal (e.g., human, rat, mouse, guinea pig, rabbit, sheep, pig, cattle, horse, cat, dog, and monkey), for example, via insertion using a catheter, direct injection into the heart, intravenous injection, or intravenous infusion.

Dose and administration frequency are not particularly limited, as long as the effects of the present invention can be obtained. The dose represented by the number of cells per administration for an adult generally ranges from approximately 10⁴ to 10⁸ cells, and preferably ranges from approximately 10⁵ to 10⁷ cells. Any administration frequency can be employed, as long as it is 1 instance or more. In general, administration frequency ranges from 1 to 10 instances and preferably approximately ranges from 1 to 5 instances.

### Example

### (A) Experimental methods

### (1) A model for inducing differentiation into myocardial cells using pluripotent P19CL6 cells

Undifferentiated P19CL6 cells were put into bacteria culture dishes via "hand drop" and then cultured in the presence of 1 % dimethyl sulfoxide. After approximately 4 days of culture, medium was exchanged every 2 days and then the cells were further cultured. A phenomenon appears such that the thus cultured cells form masses that pulsate autonomously. Specifically, 4 × 10⁵ cells were put into a 60-mm bacteria culture dish via "hand drop" (in the presence of 1% dimethyl sulfoxide). Autonomously pulsating myocardial cell populations were formed by adhesion culture. The time at which autonomous pulsation appeared and autonomous pulsation frequency were noted.

### (2) Transfection of TNNI3K gene

The gene pcDNA6-FLAG/TNNI3K of a TNNI3K molecule, which is a novel MAP kinase, was introduced, so that a cell line expressing TNNI3K at a high level was prepared by the following method. TNNI3K gene pcDNA6-FLAG/TNNI3K was introduced into P19CL6 cells using an electroporation method (electropoie 2000, Invitrogen), thereby preparing a blasticidin (Invitrogen) (500 µg/ml) resistant stable cell line expressing TNNI3K at a high level. The TNNI3K expression levels in a TNNI3K cell group and a Flag-only cell group composed of undifferentiated cells and differentiated and mature cells (on day 16) were confirmed using a Western blot method. Hence, cell groups expressing TNNI3K at high levels were obtained.

### (3) Observation of the number of pulsating myocardial cell populations and the time at which pulsation appeared

The number of pulsating myocardial cell populations, the time at which pulsation appeared, and pulsation frequency in culture dishes were observed. The results were compared with those in the Flag-only group and in cell groups expressing TNNI3K at high levels.

Spontaneous pulsation of single myocardial cells was observed via a whole-cell patch-clamp method using an EPC-7 patch clamp amp (List Electronic). A patch electrode was prepared using a PB-7 puller (Narishige Corp. LTD) and had resistance between 2 and 6 MΩ. Voltage-clamp pulse generation and data acquisition were controlled and analyzed using a PC computer provided with pCLAM software (Axon Instrument). Recording was performed at 3 Hz, followed by filtration. Whole-cell leakage and currents were subtracted online using a P/4 procedure. The spontaneous action potential of myocardial cells during the differentiation period was recorded using current clamp technology at room temperature.

### (4) Morphological test on pulsating myocardial cells

The shapes and the amounts of positive cells immuno-stained with an α-actinin antibody were observed. Furthermore, α-actinin protein expression was compared by Western blotting (α-actinin expression is a first index for indicating differentiation into myocardial cells). Specifically, a test was conducted as follows.

For the morphological test, differentiated cells derived from the heart were stained with a monoclonal antibody (Sigma) against α-actinin, followed by analysis using a confocal microscope. Specifically, cells were harvested, fixed in a 35 mm dish using acetone/methanol (1:1) for 2 minutes, washed, and then subjected to blocking using 3% BSA/PBS for 15 minutes. Subsequently, a primary antibody (anti-α-actinin antibody diluted with 0.2% Triton X-100/PBS) was added for 1 hour. Next, a secondary antibody (FITC-labeled goat anti-mouse IgG) diluted with 0.2 % BSA/PBS was added. The resultant was washed, and then fixed with 2.5% DABCO/80% glycerol. Confocal microscopic photographs were obtained using a confocal microscope (Fluoview, FV300, OLYMPUS CORPORATION) and then analyzed.

Western blotting was performed as follows. Proteins were prepared from the myocardial cells (at different differentiation stages of the Flag-only group and of the groups expressing TNNI3K at high levels). These cells were harvested, cultured for 24 hours, harvested, and then lysed. Proteins were then separated by 15% polyacrylamide gel electrophoresis for 2 hours and then transferred to nitrocellulose membranes by electroblot for 3 hours. The membranes were subjected to blocking with skim milk overnight, washed, and then incubated with anti-pcDNA-flag/TNNI3K (fowl anti-human, 1:1000), anti-α-actinin (rabbit anti-mouse, 1:800), or anti-Bax (rabbit anti-mouse, 1:500). Subsequently, the membranes were washed and then incubated with an HRP-labeled secondary antibody (1:3000 to 1:5000) at room temperature for 1 hour. Specific proteins were detected using ECL (Amersham).

### (5) Experiment by whole-cell patch-clamp method using single myocardial cells isolated using an enzyme (collagenase)

The spontaneous action potential was recorded. The sensitivity of such action potential to epinephrine, which is an adrenaline receptor agonist, was observed.

With the use of single myocardial cells loaded with Fura-2/AM, the effect of caffeine, ryanodine, or a high-K⁺ solution on intracellular Ca²⁺ concentration was studied. For measurement of intracellular Ca²⁺ concentration, single myocardial cells were isolated using collagenase (1.5 mg/ml), placed on a mount coverslip dish (35 mm in diameter) coated with poly-L-lysine, and then incubated with 3.5 µM fura-2/AM (DOJINDO LABORATORIES) at 37°C for 45 minutes. Subsequently, the cells were washed 4 times with medium. Micro fluorescence intensity measurement was performed at room temperature in physiological saline (140 mM NaCl, 5 mM KCI, 1.5 mM CaCl₂, 1 mM MgCl₂, 10 mM Hepes, 10 mM glucose, and pH 7.4). Images of the cells with an image ratio of 340 nm/380 nm were collected using an image processor system (ACOUS cosmos, Hamamatsu Photonics K.K.) and then stored on an analysis MO disk. Moreover, a Ca²⁺ response in the presence of a high K⁺ solution (142 mM), caffeine (10 mM), or ryanodine (2.6 µM) was also examined.

### (6) Studies on the relationship among TNNI3K, myocardial cell development, and suppression of apoptosis during maturation phase

Cells were treated with heat at 45°C for 90 minutes, cultured, and then stained with an annexin-V antibody. The percentage of annexin-V positive cells was calculated by a FACScan method.

Furthermore, Bax protein expression was analyzed using the Western blot method. Specifically, cells were irradiated with UV for 20 seconds, cultured for 24 hours, harvested, lysed, and then Western-blotted.

### (7) The effect of TNNI3K on a myocardial infarction model established via ligation of the left anterior descending coronary artery (LAD) of rat hearts

The left anterior descending coronary artery (LAD) of each rat (male Spague-Dawley rat) (body weight ranging from 275 g to 320 g and 8 to 10 weeks old) was ligated, thereby establishing a myocardial infarction model. The rats were divided into the following 3 groups: (i) a group of rats to which only medium was administered (n = 9), (ii) a group of rats to which P19CL6 was administered via intramyocardial administration (n=5), and (iii) a group of rats to which P19CL6 cells expressing TNNI3K at high levels (n=7) were administered. P19CL6-derived myocardial cells (4×10⁶ cells/site, x6 positions) at an early phase of differentiation (day 4) which express TNNI3K at high levels were implanted into locations with myocardial infarction. Myocardial infarction sizes (including infracted myocardial thickness, the ratio of infracted myocardial thickness to non-infarcted myocardial thickness, left ventricular diameter, and enlargement index) and conditions of cell death of myocardial cells (necrosis or apoptosis) were observed. The results were compared with those in the case of the heart into which medium alone had been introduced or into which P19CL6-derived myocardial cells at an early phase of differentiation (day 4) into which no TNNI3K had been introduced had been implanted.

### (B) Results

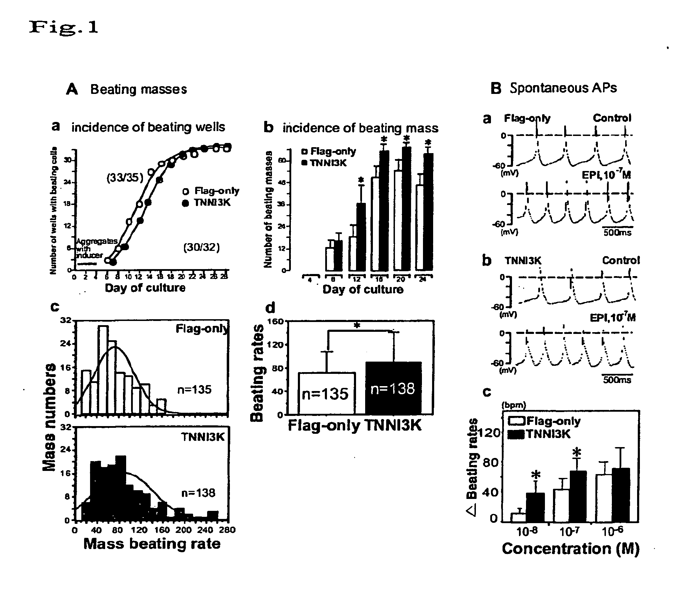

### (1) TNNI3K promotes the formation of pulsating cell groups at an early development phase of myocardial cells and promotes increased pulsation frequency (Fig. 1 A)

In the results of this experiment, pulsation of P19CL6-cell-derived myocardial cells was observed on day 6 after the start of differentiation induction in the earliest case. On and after day 15, pulsation was observed for most masses. In both cases including those of Flag-only cells and cells expressing TNNI3K at high levels, pulsating myocardial cell populations were formed. In the case of the cells expressing TNNI3K at high levels, the number of myocardial cell populations was greater than that in the case of Flag-only. The mean pulsation frequency was approximately 70 times/minute (71.3 ± 36.1, n = 135 masses) in the case of Flag-only. In the case of the myocardial cells derived from the cells expressing TNNI3K at high levels, the mean pulsation frequency was approximately 90 times/minutes (88.9 ± 52.7, n = 138 masses), indicating a significant increase compared with that in the case of Flag-only (Fig. 1A).

### (2) TNNI3K enhances the sensitivity of the spontaneous action potential of single myocardial cells to epinephrine

Pulsation frequency resulting from the spontaneous action potential of single myocardial cells was increased in a manner that depended on epinephrine concentrations (10⁻⁸, 10⁻⁷, and 10⁻⁶M). TNNI3K significantly enhanced the sensitivity of the myocardial cells to administration of epinephrine at low concentrations. These results demonstrate that TNNI3K promotes the development of autonomic modulation of such as sympathetic nerve at early development and differentiation phases of myocardial cells (Fig. 1B).

### (3) TNNI3K increases the number of positive cells immuno-stained with an α-actinin antibody and the expression level of α-actinin protein

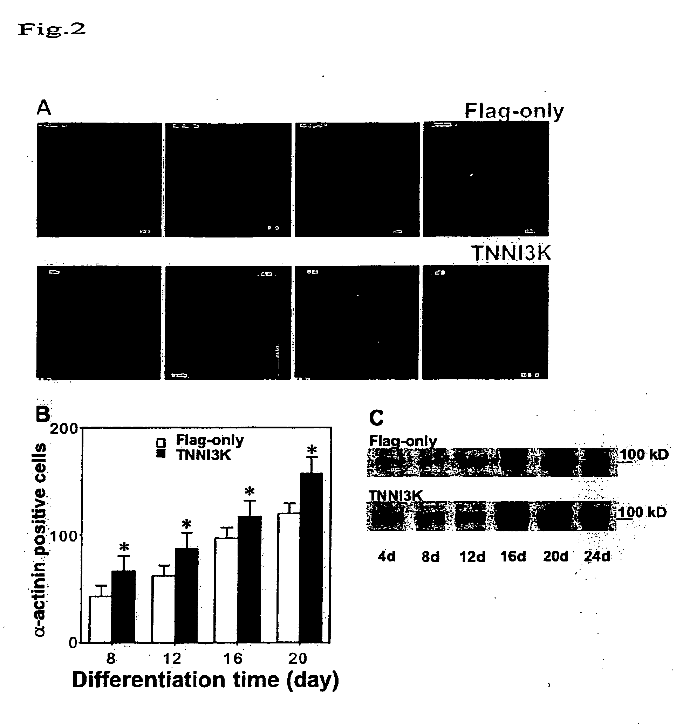

When TNNI3K was compared with Flag-only, there were no differences in terms of size and shape of myocardial cells that had been formed by differentiation of progenitor cells. However, in terms of the number of positive cells immuno-stained with the α-actinin antibody and the expression level of α-actinin protein, the number and the level were significantly higher in the case of the cells expressing TNNI3K at high levels than in the case of Flag-only (Fig. 2A, B, and C).

### (4) TNNI3K significantly suppresses the occurrence of apoptosis at an early differentiation phase of P19CL6-derived myocardial cells

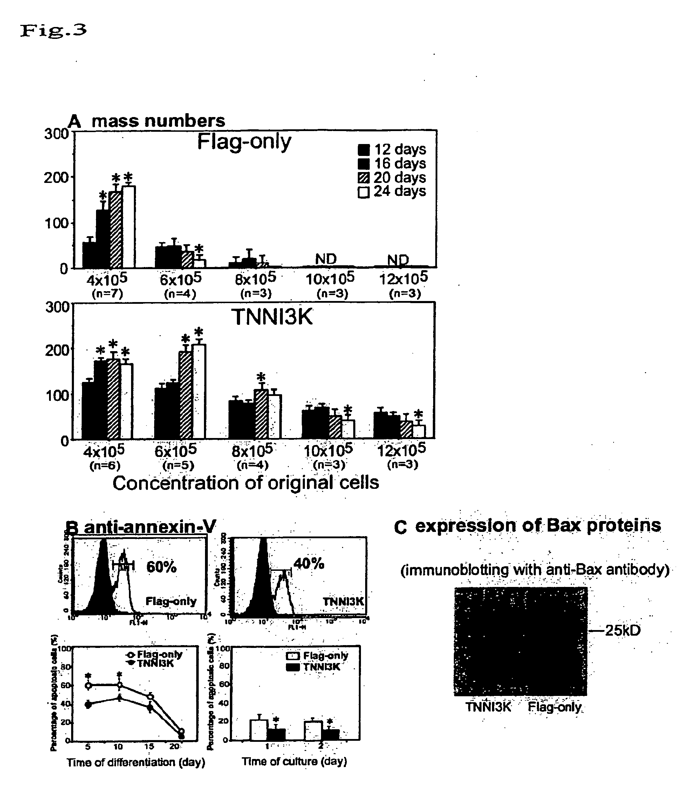

Cells on day 5 after differentiation were treated at 45°C for 90 minutes and then stained with an annexin-V antibody. The percentages of annexin-V positive cells were examined. As a result, it was confirmed that the percentage of annexin-V positive cells in the case of cells expressing TNNI3K at high levels was lower than the same in the case of Flag-only (Fig. 3B).

Bax expression analysis: Cells were irradiated with UV for 20 seconds and then cultured for 24 hours. Bax expression level was then analyzed by the Western blot method. As a result, compared with the Flag-only group, Bax protein expression level at an early differentiation phase was significantly decreased in the case of cells expressing TNNI3K at high levels (Fig. 3C).

### (5) The effect of TNNI3K on intracellular Ca²⁺ response in P19CL6-cell-derived myocardial cells

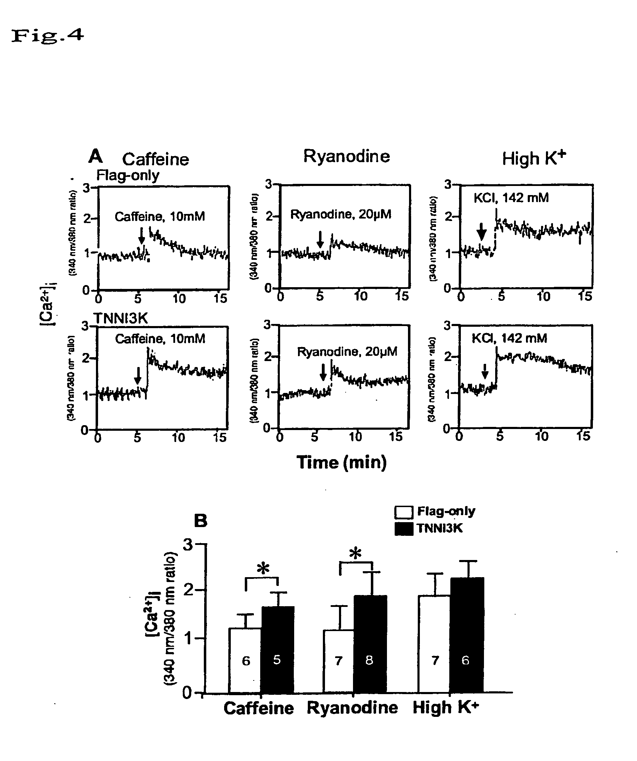

The effect of caffeine, ryanodine, or a high-K⁺ solution on intracellular Ca²⁺ concentration was studied in P19CL6-cell-derived myocardial cells loaded with Fura-2/AM. The degree of increases in Ca²⁺ concentration (induced by a drug for promoting release from intracellular Ca²⁺ store, caffeine (10 mM), or ryanodine (2.6 µM) was stronger in the group expressing TNNI3K at high levels than in the Flag-only group. When Ca²⁺ channels existing in cell membranes were activated via administration of the high-K⁺ solution, continuous increases in intracellular Ca²⁺ concentration were induced to almost the same degree in both groups (Fig. 4).

### (6) TNNI3K improves left ventricular remodeling in a myocardial infarction model in an in vivo experiment

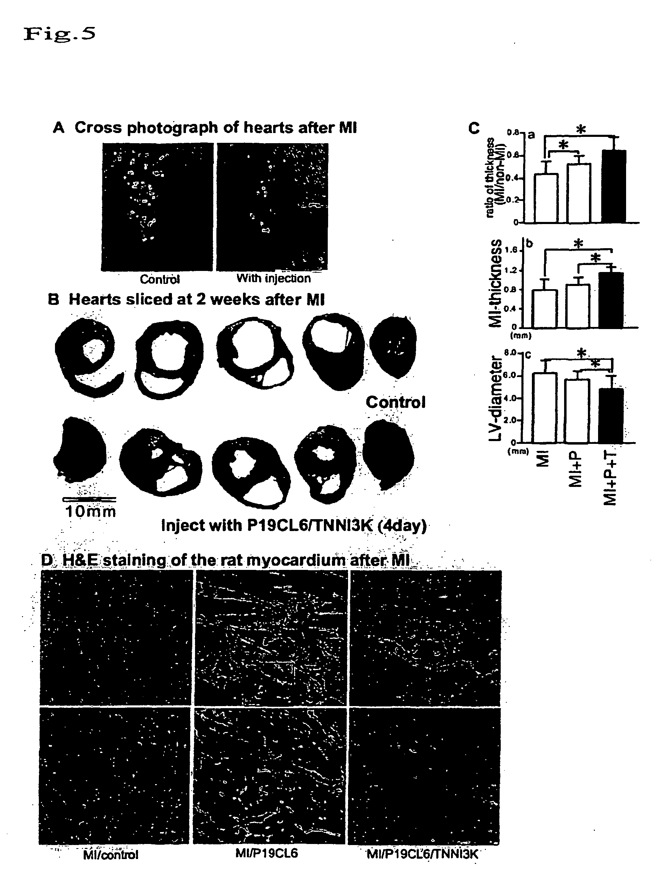

In an *in vivo* experiment using an SD rat myocardial infarction model, P19CL6 cells at an early differentiation phase which express TNNI3K at high levels were injected into an infracted myocardial location. As a result, improvement in left ventricular remodeling in the infracted heart was promoted as compared with the case where P19CL6 fat alone was injected (Fig. 5).

Furthermore, P19CL6 cells at an early differentiation phase which express TNNI3K at high levels were injected. In this case, more decreased myocardial infarction sizes and more suppressed apoptosis at infracted locations were observed, as compared with the case where only P19CL6 cells were injected (Fig. 5).

### Industrial Applicability

According to the present invention, the TNNI3K gene is introduced to achieve increased TNNI3K protein levels, regeneration of pathological cardiac muscles, and cardiomuscular functional recovery. Thus, novel therapeutic strategies against cardiomyopathy and heart malfunction can be provided. Specifically, implantation of myocardial progenitor cells expressing MAP kinase TNNI3K at high levels into a location affected with myocardial ischemia or local myocardial infarction makes it possible to promote myocardial cell regeneration, reduce the infracted area, and improve left ventricular remodeling. The technology of the present invention is characterized by simple procedures, high specificity, and significant therapeutic effects.

### Brief Description of the Drawings

Fig. 1 shows spontaneous pulsation in cells excessively expressing TNNI3K. Fig. A shows spontaneous pulsation of P19CL6-derived heart masses.
   a: The number of spontaneously pulsating masses formed in a 60-mm culture well. This figure shows an image at the time when the first pulsating masses were formed.
   b: The number of pulsating masses formed. The number of pulsating masses was counted under a microscope.
   c and d: Mean pulsation frequency of pulsating masses.
Fig. B shows the response of spontaneous action force to epinephrine. Such spontaneous action force was recorded by the whole-cell patch-clamp method using single myocardial cells, thereby allowing examination of responses to epinephrine.
   a: Typical example of Flag-only cells.
   b: Typical example of TNNI3K-expressing cells.
   c: Dose dependency of increases in the number of pulsations upon stimulation with EP1
Fig. 2 shows the results of immunohistological analysis conducted on single myocardial cells.
   A: Typical photographs showing single myocardial cells at the final phase of differentiation (day 16) in the Flag-only group and the TNNI3K group.
   B: The number of α-actinin positive cells. In both the Flag-only group and the TNNI3K group, the number of actinin positive cells was increased in a manner that depended on days of culture.
   C: The results of Western blot performed for α-actinin expression. Days of differentiation are shown in the lower case of each lane.
Fig. 3 shows inhibition of apoptosis during myocardial development by excessive expression of TNNI3K.
   A: The effect of changes in the number of first cultured cells on the development of myocardial cells. The number of the first cells was varied. The mass formation by myocardial cells was then examined in the Flag-only group and in the TNNI3K group.
   B: Proportion of annexin V positive cells as measured by FACScan. Graphs in the lower column show the proportions of apoptotic cells in the cells during differentiation (left graph) and undifferentiated (right graph) cells with time course.
   C: Bax protein expression in myocardial cells.
Fig. 4 shows the effect of TNNI3K to enhance intracellular calcium responses in P19CL6-derived myocardial cells.
   A: Transient increases in intracellular calcium concentration by administration of caffeine, ryanodine, or high-K.
   B: Summary of intracellular calcium response in the Flag-only group and the same in TNNI3K.
Fig. 5 shows the *in vivo* effect of the intramyocardial injection of P19CL6 cells expressing TNNI3K at high levels on myocardial infarction.
   A: Photograph of a heart section.
   B: Photograph of the horizontally cut heart.
   C: The ratio of the wall thickness of the cardiac ventricle in a region with myocardial infarction (MI) to the same in a region without myocardial infarction (MI), MI thickness (the wall thickness of the cardiac ventricle in a myocardial infarction region, and medium group), and LV diameter (left ventricle diameter).
      MI: Myocardial infarction
      MI+P: Myocardial infarction + injection of P19CL6 cells
      MI+P+T: Myocardial infarction + injection of differentiated P19CL6 cells expressing TNNI3K at high levels.
   D: HE staining of the sections obtained from rat cardiac muscle (on day 14 after myocardial infarction).

## Claims

1. A pharmaceutical composition for the treatment of myocardial ischemia or myocardial infarction, which comprises a myocardial progenitor cell expressing MAP kinase TNNI3K at a high level as an active ingredient, wherein said myocardial progenitor cell is a myocardial progenitor cell into which an expression vector for expressing MAP kinase TNNI3K at a high level is introduced, with the provision that human embryonic cells are excluded.

2. The pharmaceutical composition according to claim 1, wherein the myocardial progenitor cell is an undifferentiated myocardial cell.

3. The pharmaceutical composition according to claim 1 or 2, wherein the myocardial progenitor cell is an undifferentiated P19CL6 cell.

4. The pharmaceutical composition according to any of claims 1 to 3 for administration to a myocardial location with ischemia or infarction.

## Patentansprüche

1. Eine pharmazeutische Zusammensetzung zur Verwendung in der Behandlung von Myokardischämie oder Myokardinfarkt, enthaltend eine Myokardvorläuferzelle als Wirkstoff, die MAP-Kinase TNNI3K auf hohem Niveau exprimiert, wobei die Myokardvorläuferzelle eine Myokardvorläuferzelle ist, in die ein Expressionsvektor zur Expression der MAP-Kinase TNNI3K auf hohem Niveau eingeführt wurde, mit der Maßgabe, dass menschliche embryonale Zellen ausgeschlossen sind.

2. Die pharmazeutische Zusammensetzung gemäß Anspruch 1, wobei die Myokardvorläuferzelle eine undifferenzierte Myokardzelle ist.

3. Die pharmazeutische Zusammensetzung gemäß Anspruch 1 oder 2, wobei die Myokardvorläuferzelle eine undifferenzierte P19CL6-Zelle ist.

4. Die pharmazeutische Zusammensetzung gemäß irgend einem der Ansprüche 1 bis 3 zur Verabreichung in einen von Ischämie oder Infarkt betroffenen Bereich des Myokards.

## Revendications

1. Composition pharmaceutique destinée au traitement d'une ischémie myocardique ou d'un infarctus du myocarde, qui comprend une cellule progénitrice myocardique exprimant la MAP kinase TNNI3K à un niveau élevé en tant que principe actif, où ladite cellule progénitrice myocardique est une cellule progénitrice myocardique dans laquelle un vecteur d'expression pour exprimer la MAP kinase TNNI3K à un niveau élevé est introduit, à condition que les cellules embryonnaires humaines soient exclues.

2. Composition pharmaceutique selon la revendication 1, dans laquelle la cellule progénitrice myocardique est une cellule myocardique indifférenciée.

3. Composition pharmaceutique selon la revendication 1 ou 2, dans laquelle la cellule progénitrice myocardique est une cellule P19CL6 indifférenciée.

4. Composition pharmaceutique selon l'une quelconque des revendications 1 à 3, pour une administration à un emplacement myocardique affecté par une ischémie ou un infarctus.
